# NOUVEAU FASCICULE DE BREVET EUROPEEN

(11) **EP 0 872 240 B2**
(45) Date de publication et mention de la décision concernant l'opposition: **10.09.2008**
(45) Mention de la délivrance du brevet: 24.07.2002
(21) Numéro de dépôt: 98400932.4
(22) Date de dépôt: 15.04.1998
(51) Int. Cl.: A61K 33/06, A61K 9/00, A61P 19/10

(54) **Préparation solide vitamino-calcique, son procédé d'obtention et son utilisation**
Feste Arzneizubereitung aus Vitaminen und Kalzium, Verfahren zu ihrer Herstellung und ihre Anwendung
Solid preparation from vitamins and calcium, its manufacturing process and use

(30) Priorité: 16.04.1997 FR 9704703
(43) Date de publication de la demande: 21.10.1998
(73) Titulaire: LABORATOIRES BESINS INTERNATIONAL, 75003 Paris (FR)
(72) Inventeur: Salin-Drouin, Dominique, 91370 Verrieres Le Buisson (FR)
(74) Mandataire: Boulinguiez, Didier

(56) Documents cités:
- WO-A-96/09036
- GB-A- 2 304 285
- DATABASE WPI Week 9740 Derwent Publications Ltd., London, GB; AN 97-431398 [40] XP002051240 & JP 09 194 381 A (KOWA) 29 juillet 1997
- Monographie "IDEOS comprimées à croquer" de la base de données BIAM
- Monographie "IDEOS" du Dictionnaire Vidal, édition 1996

## Description

La présente invention a pour objet une préparation solide vitamino-calcique, utile notamment pour prévenir et combattre l'ostéoporose.

Elle a également pour objet le procédé de fabrication de cette préparation.

Les sels de calcium sont largement utilisés en pratique médicale et leur consommation va en augmentant avec le développement des campagnes d'information sur la prévention de l'ostéoporose. Les apports recommandés chez l'adulte, susceptibles de protéger 95 % de la population d'une balance calcique négative, sont estimés à 800-900 mg par jour. Cependant, les enquêtes alimentaires montrent qu'une grande partie de la population a une consommation quotidienne de calcium inférieure à ces recommandations. Ainsi, après l'âge de 35 ans, plus de 75 % des femmes américaines auraient un apport calcique alimentaire insuffisant. Chez l'adolescent, entre 10 et 19 ans, et chez les femmes lors de la grossesse et de la lactation, les besoins calciques augmentent et les apports recommandés sont alors de l'ordre de 1200 à 1400 mg par jour. De même, les besoins calciques augmentent chez la femme après la ménopause et sont de l'ordre de 1500 mg pour le maintien d'une balance calcique équilibrée.

L'une des principales indications de la calcithérapie est la prévention de l'ostéoporose. L'administration d'un supplément calcique oral permettrait en effet de réduire l'accélération de la perte osseuse qui suit la ménopause. Les apports calciques chez l'adolescent et l'adulte jeune pourraient également contribuer à augmenter le pic de masse osseuse acquis à la fin de la croissance.

Il est connu que la vitamine D, administrée en association avec le calcium, permet d'augmenter l'absorption de celui-ci dans l'organisme. Les mécanismes par lesquels la vitamine D agit pour maintenir des concentrations normales de Ca²⁺ dans le plasma sont de faciliter son absorption dans l'intestin grêle, d'augmenter sa mobilisation dans les os, et de diminuer son excrétion par les reins. Ces processus servent à maintenir le calcium à des concentrations plasmatiques qui sont essentielles pour une bonne minéralisation des os, une activité neuro musculaire normale, et d'autres fonctions dépendantes du Ca²⁺. Il est ainsi désormais parfaitement admis que la vitamine D exerce des effets directs et indirects sur les cellules qui sont responsables de la reconstitution des os.

En ce qui concerne plus particulièrement le sujet âgé, il a été démontré que l'administration d'un supplément calcique permet, en association à des doses faibles de vitamine D, de freiner l'hyperparathyroïdisme secondaire fréquemment observé dans cette situation et pouvant malheureusement contribuer à la survenue de fractures du col fémoral.

Ces indications expliquent l'intérêt qui est actuellement porté aux associations de sels de calcium et de vitamine D, plus particulièrement pour la prévention de l'ostéoporose.

Ces associations comportent le plus généralement un fort dosage de calcium pour un faible dosage de vitamine D. Si la vitamine D est en effet très bien absorbée par l'organisme, ce n'est pas le cas du calcium, qu'il est donc nécessaire d'absorber en quantités élevées pour maintenir une concentration efficace.

La demande de brevet français n° 2 724 844 décrit ainsi une association vitamino calcique comprenant du calcium et de la vitamine D, cette association comprenant nécessairement, pour sa mise en oeuvre, un liant à sec et en milieu humide, combiné en quantité synergique avec au moins un diluant, au moins un liant et au moins un lubrifiant, l'un au moins dudit liant et dudit lubrifiant étant un édulcorant. Le procédé d'obtention de l'association ainsi décrite consiste (a) à granuler le calcium sous forme élémentaire avec du liant à sec et en milieu humide ; (b) à prémélanger la vitamine D avec le liant édulcorant dans une étape séparée ; (c) à mélanger dans une autre étape séparée le liant édulcorant, du liant édulcorant supplémentaire et l'arôme avec les produits des étapes (a) et (b) tout en ajoutant du lubrifiant ; (d) à comprimer éventuellement le mélange sur presse rotative.

Il est indiqué de façon on ne peut plus claire dans cette demande de brevet que les contraintes des principes actifs ne permettent pas une mise en oeuvre directe, et que des associations vitamino-calciques du genre de celles revendiquées dans la présente demande ne peuvent être fabriquées par compression directe.

Or il est du mérite de la Société Demanderesse d'avoir trouvé, en dépit de ce préjugé hautement défavorable, qu'il est possible de fabriquer par compression directe une préparation solide vitamino-calcique comprenant du calcium-élément et au moins une vitamine D, ladite préparation répondant à tous les desiderata de la technique, notamment du point de vue de l'homogénéité et de la stabilité, et présentant une acceptabilité chez les patients tout à fait remarquable, en raison notamment du choix d'une texture particulière.

La préparation solide vitamino-calcique comprenant du calcium et au moins une vitamine D conforme à l'invention est caractérisée par le fait qu'elle se présente sous la forme d'un comprimé à croquer obtenu par compression directe et ayant une dureté comprise entre 75 et 300 N, de préférence entre 85 et 275 N, et plus préférentiellement encore entre 100 et 250 N et qu'elle contient de 1 à 3 %, de préférence environ 2 % de lubrifiant, celui-ci étant préférentiellement le stéarate de magnésium.

Le comprimé conforme à l'invention comprend au moins un sel de calcium, au moins une vitamine D, au moins un excipient de compression directe et au moins un lubrifiant. Il contient le plus souvent également un ou plusieurs arômes, un ou plusieurs colorants, ainsi qu'éventuellement un ou plusieurs édulcorants intenses et un ou plusieurs agents d'écoulement.

Le comprimé selon l'invention présente une quantité de sel de calcium généralement comprise entre 500 et 2500 mg, de préférence entre 750 et 2000 mg, et plus préférentiellement encore entre 1000 et 1500 mg. Suivant la nature du sel de calcium utilisé, la quantité de calcium-élément se trouve de ce fait comprise entre environ 100 et environ 1000 mg de calcium-élément (les posologies préconisées sont presque toujours exprimées en équivalent de calcium-élément).

La quantité de vitamine D que comporte le comprimé se situe quant à elle entre 50 et 1000 U.I (unités internationales), de préférence entre 100 et 800 UI et plus préférentiellement encore entre 250 et 600 UI.

La masse restante du comprimé est essentiellement constituée d'un ou plusieurs excipients de compression directe, le poids total d'un comprimé se situant entre 1000 et 5000 mg, de préférence entre 1500 et 3500 mg et plus préférentiellement encore entre 2000 et 3000 mg.

Le sel de calcium entrant dans la composition vitamino-calcique conforme à l'invention est choisi parmi le carbonate de calcium, le chlorure de calcium, le lactate de calcium, le gluconate de calcium, le glucoheptonate de calcium, le gluconolactate de calcium, le citrate de calcium, le pédolate de calcium, le glycérophosphate de calcium, le phosphate de calcium ainsi que leurs mélanges. On peut également utiliser de l'hydroxyapatite micro cristalline.

Le carbonate de calcium constitue le sel préféré.

La vitamine D est choisie parmi la vitamine D2, ou ergocalciférol, ou la vitamine D3, ou cholécalciférol, ainsi que leurs mélanges. La vitamine D3 est préférée et on l'utilise préférentiellement sous une forme pulvérulente incorporée dans une matrice qui augmente sa stabilité, notamment sous une forme pulvérulente qui se présente sous forme de billes et dont l'enrobage est constitué par de la gélatine, la teneur en vitamine D3 étant alors de 100 000 UI par gramme de poudre.

L'excipient de compression est préférentiellement choisi parmi les polyols tels que le sorbitol, le xylitol, le mannitol ou le maltitol, ces polyols ayant été rendus directement compressibles par des traitements physicochimiques appropriés ou présentant une qualité sélectionnée pour offrir des propriétés de compressibilité satisfaisantes. Outre le fait qu'ils présentent de bonnes aptitudes technologiques en ce qui concerne la compression directe, ces polyols ou sucres-alcools ont l'énorme avantage de présenter un goût sucré tout en étant acariogènes et moins caloriques que le saccharose.

Afin d'obtenir la texture désirée pour le produit conforme à l'invention, on utilise en particulier du sorbitol et du xylitol. Le sorbitol constitue de préférence de 55 % à 90 % de l'excipient de compression directe utilisé dans la formulation du comprimé selon l'invention, le xylitol en constituant de 10 % à 45 %. Des qualités de sorbitol ou de xylitol particulièrement adaptées pour la compression peuvent êtres trouvées en particulier auprès des Sociétés ROQUETTE et FINNSUGAR, notamment sous les marques NEOSORB® P 60 W et XYLITAB®.

Le lubrifiant peut être choisi parmi l'acide stéarique, les huiles de ricin ou de coton hydrogénées, le béhénate de glycérol et le stéarate de magnésium, ce dernier étant préféré. Des taux de 1 % à 3 %, de préférence voisins de 2 %, sont utilisés.

On peut utiliser, pour aromatiser le comprimé, les arômes ou mélanges d'arômes que l'on rencontre habituellement sur le marché. Selon une caractéristique préférentielle de l'invention, on utilise cependant un arôme cocktail de fruits tel que celui commercialisé sous la référence 34 A 016 par la Société IFF. Les multiples essais réalisés au cours de l'étude et de développement de la formulation conforme à l'invention ont en effet démontré que cet arôme était particulièrement bien adapté à la nature et aux quantités des produits utilisés dans ladite formulation : notamment carbonate de calcium, sorbitol en quantité prépondérante, et xylitol.

Des quantités minimes d'édulcorants artificiels tels que l'aspartame ou l'acésulfame K peuvent également être utilisées avantageusement dans la formule. Enfin, des additifs tels que la silice colloïdale anhydre (agissant comme agent d'écoulement) ou des colorants peuvent compléter la formulation.

Non seulement la Société Demanderesse a eu le mérite, en dépit des préjugés techniques contraires, de montrer qu'il était possible de préparer par compression directe des comprimés calcium-vitamine D, mais elle a eu également le mérite de montrer que ces comprimés, obtenus par compression directe, présentaient une acceptabilité extrêmement élevée dès lors que leur texture était choisie dans une gamme de propriétés donnée, ladite gamme étant en fait essentiellement traduite et caractérisée par une dureté des comprimés comprise entre 75 et 300 N, de préférence entre 85 et 275 N, et plus préférentiellement encore entre 100 et 250 N.

Ainsi, grâce au choix du procédé de compression directe et à la sélection d'une texture particulière, caractérisée par une dureté comprise entre les valeurs précitées, les comprimés conformes à l'invention se sont révélés présenter d'excellentes performances dans des études d'acceptabilité effectuées en double aveugle.

Ils sont en effet considérés comme ayant un goût "bon" et "très bon" dans plus de 70 % des réponses, et "faciles" et "très faciles" à croquer dans 73 % des réponses. Les comprimés sont également très bien tolérés (40 %) ou bien tolérés (37 %). De même, à l'issue d'un traitement de 15 jours consistant à prendre un comprimé de 2.5 g (dosé à 1.250 g de carbonate de calcium et 400 UI de vitamine D3) le matin et le soir avant chaque repas avec un verre d'eau, les comprimés devant être croqués, l'impression générale du traitement a été considérée comme "bonne" ou "très bonne" dans 73 % des cas.

En fait, les tests d'évaluation gustative indiquent que les comprimés selon l'invention se caractérisent par une texture peu fondante, très croquante, peu collante aux dents, et assez persistante en bouche.

La dureté du comprimé selon l'invention est mesurée selon une technique tout à fait conventionnelle, consistant à mesurer la résistance à la rupture des comprimés (cf Pharmacopée Européenne - 1977, p 135). Cette résistance est typiquement mesurée sur des comprimés d'un poids unitaire d'environ 2.500 mg, d'une épaisseur d'environ 4.50 mm. La dureté est mesurée à l'aide d'un duromètre SCHLEUNIGER 2E, commercialisé en France par les Etablissements FROGERAIS, et elle est exprimée en newtons, en prenant la valeur moyenne mesurée sur 10 comprimés.

Le procédé de fabrication de la préparation solide vitamino-calcique selon l'invention consiste à peser les différents composants de la préparation, à les mélanger pendant un temps suffisant pour assurer une bonne homogénéisation, à effectuer la compression du mélange obtenu, comportant le lubrifiant, à l'aide d'une presse rotative ou alternative, et à conditionner les comprimés obtenus. Les paramètres de compression sont choisis de manière à ce que la dureté des comprimés soit comprise entre 75 et 300 N, de préférence entre 85 et 275 N et plus préférentiellement encore entre 100 et 250 N.

De préférence, on effectue tout d'abord, pendant environ 15 minutes, un prémélange de la vitamine D3 avec l'un des excipients de compression directe, comme le xylitol par exemple, et on ajoute un agent d'écoulement, notamment la silice colloïdale.

On effectue ensuite un léger tamisage sur tamis de 1000 µm, et on procède au mélange total en ajoutant le carbonate de calcium, le reste des excipients de compression directe, l'édulcorant intense, le ou les arômes. Le tout est mélangé pendant une vingtaine de minutes environ sur un mélangeur de type TURBULA par exemple, à une vitesse d'environ 60 tours/min. On ajoute ensuite le lubrifiant, préférentiellement constitué par le stéarate de magnésium, et on mélange de nouveau pendant une durée d'environ 3 minutes à une vitesse de 60 tours/minute. Le mélange final ainsi obtenu est alors approvisionné sur les machines de compression réglées de manière à conduire au poids et à la dureté de comprimés voulus, ces deux paramètres étant contrôlés au cours de la fabrication. Le produit est ensuite conditionné.

Le procédé conforme à l'invention présente, par rapport notamment au procédé décrit dans la demande de brevet français n° 2 724 844, l'avantage d'être beaucoup plus simple et plus économique.

Contrairement à ce procédé de l'art antérieur, il ne nécessite en effet aucun traitement préalable de la poudre, et ne nécessite en particulier aucune adjonction de liquide. Il fait appel à beaucoup moins de produits et se réalise en beaucoup moins d'étapes.

Selon une réalisation particulièrement avantageuse, la préparation solide vitamino-calcique conforme à l'invention se présente sous la forme d'un comprimé sécable obtenu par compression directe et ayant la composition type suivante :
- carbonate de calcium : 1100 - 1350 mg
- cholécalciférol (sous la forme de vitamine D3 enrobée) : 350 - 450 UI (3,5 - 4,5 mg)
- sorbitol : 750 - 1000 mg
- xylitol : 180 - 350 mg
- Aspartame : 4 - 8 mg
- Silice colloïdale anhydre : 8-15 mg
- Arôme : 40 - 70 mg
- Stéarate de magnésium : 45 - 80 mg.

On dispose ainsi, grâce à l'invention, d'une préparation vitamino-calcique extrêmement bien acceptée par les patients, les personnes âgées notamment, et donc extrêmement utile dans les traitements de prévention de l'ostéoporose.

L'invention pourra être mieux comprise à l'aide de l'exemple de réalisation suivant, qui est donné à titre purement illustratif.

### EXEMPLE :

875 kg de prémélange sont préparés, destinés à la fabrication d'environ 350.000 comprimés d'un poids unitaire de 2.500 mg.

La formulation est la suivante :
- carbonate de calcium : 437,1500 kg
- vitamine D3 : 1,4000 kg
- sorbitol (NEOSORB® P60W) : 303,0125 kg
- xylitol : 87,5000 kg
- aspartame : 2,1875 kg
- arôme cocktail de fruits (IFF 34 A 016) : 17,5000 kg
- silice colloïdale anhydre : 4,3750 kg
- stéarate de magnésium : 21,8750 kg.

Les différents composants de cette formulation sont pesés soigneusement sur balance de pesée METLER et multirange.

Un prémélange du xylitol, de la vitamine D3, de la silice colloïdale anhydre est tout d'abord réalisé dans un mélangeur de type planétaire ou type Paterson, mélangeur à chute libre en Y d'un volume utile de 600 litres, la vitesse de mélange étant de 1 tour/minute.

Après un temps de mélange de 15 minutes, suffisant pour assurer une bonne homogénéité de la masse, le prémélange obtenu est tamisé sur un tamis de 1000 micromètres d'ouverture de maille.

Le prémélange tamisé est alors mélangé avec les autres composants de la formulation, c'est-à-dire le carbonate de calcium, le sorbitol, l'aspartame et l'arôme cocktail de fruits, et le mélange est effectué dans un mélangeur de type Turbula, c'est-à-dire un mélangeur à chute libre de type elliptique d'un volume utile de 600 litres.

Le mélange est effectué dans ce mélangeur à une vitesse de 60 tours/minute et pendant un temps de 20 minutes. Le stéarate de magnésium est alors ajouté, toujours dans un mélangeur de type Turbula et à la même vitesse de mélange de 60 tours/minute. Après un temps de mélange de 3 minutes, le mélange final ainsi obtenu est acheminé sur une machine à comprimer de type FETTE, munie de poinçons de 20 millimètres de diamètre. Les paramètres de la machine sont réglés de façon à obtenir un poids moyen des comprimés de 2500 mg et une dureté comprise entre 150 et 170 newtons.

Au fur et à mesure de la fabrication, le poids des comprimés est vérifié et doit être situé dans une fourchette de 2,275 à 2,625 grammes. De même, le temps de délitement est mesuré et doit être inférieur ou égal à 30 minutes dans de l'eau à 37°C. L'épaisseur des comprimés obtenus est d'environ 4,50 mm et leur friabilité est de 0,45 %.

Les comprimés sont finalement conditionnés sur remplisseuse de type KING dans des tubes de polypropylène à raison de 15 comprimés par tube.

Un essai de tolérance et d'acceptabilité de ces comprimés a été effectué sur 30 personnes de sexe féminin.

L'acceptabilité a été évaluée à J15, au moyen d'échelles verbales, pour les paramètres suivants : goût du comprimé, facilité à croquer le comprimé, tolérance buccale, impression générale. Ces variables ont été évaluées au moyen d'échelles verbales en 5 points : très bon, bon, moyen, mauvais, très mauvais. Un autre paramètre : reprise de la supplémentation, a été évalué au moyen d'une échelle verbale en deux points : oui, non.

La tolérance a été évaluée par chaque personne à la fin de l'étude par une échelle verbale en deux points. Elle a également été jugée d'après la notification des effets indésirables spontanément signalés par les sujets ou observés par l'investigateur.

Les résultats de l'étude sont les suivants :

Le goût des comprimés est majoritairement considéré comme bon : 50 %. La somme des réponses "bon" (50 %) et "très bon" (20 %) représente 70 des réponses.

Le comprimé est majoritairement considéré comme facile à croquer dans 53 % des cas. La somme des réponses "facile" (53 %) et "très facile" (20 %) représente 73 % des réponses.

Au niveau buccal, dans la majorité des cas, le comprimé est très bien toléré (40 %) ou bien toléré (37 %). Seulement 10 % des personnes ont mal toléré le produit au niveau buccal.

L'impression générale est majoritairement bonne dans 60 % des cas. La somme des réponses "bon" (60 %) et "très bon" (13 %) représente 73 % des réponses. On note 13 % de réponses "mauvais" et 7 % de réponses "très mauvais". Ces réponses sont le fait de personnes ayant eu des problèmes de tolérance locale ou générale.

Enfin 70 % des sujets se sont déclarés prêts à reprendre le produit.

L'ensemble de ces réponses permet de conclure que l'acceptabilité du produit ainsi préparé est bonne, voire très bonne.

En ce qui concerne la tolérance, elle a été bonne dans 73 % des cas.

Un test d'évaluation gustative a également été réalisé sur les comprimés obtenus conformément à l'invention. Les personnes contactées pour ce test ont été sélectionnées sur une très large base. Aucun critère spécifique n'est entré en ligne de compte lors de leur sélection (âge, sexe, ostéoporose éventuelle). C'est ainsi que : l'âge est compris entre 20 et 64 ans, l'échantillon comprend des hommes et des femmes, il n'y a aucun lien de parenté entre les personnes, toutes les catégories socio-professionnelles sont représentées, aucun traitement médical particulier n'est administré.

Plusieurs critères ont été étudiés lors de la présentation du test aux personnes interrogées. C'est ainsi qu'il leur a été demandé d'apprécier la texture du comprimé (intégrant les caractéristiques : "fondant", "craquant", "collant aux dents") ainsi que le goût, essentiellement donné par l'arôme.

Les résultats de ce test sont les suivants.

En ce qui concerne le critère textural "fondant", le produit a été jugé "peu fondant" par 11 personnes, "assez fondant" par 3 personnes et "très fondant" par 2 personnes.

En ce qui concerne le critère "craquant", aucun sujet n'a jugé le produit "peu craquant", 4 sujets ont jugé le produit "assez craquant" et 12 personnes l'ont jugé "très craquant".

En ce qui concerne le critère "collage aux dents", 14 personnes ont jugé que le produit "collait peu aux dents", 1 personne a jugé qu'il "collait assez aux dents" et 1 personne a jugé qu'il "collait beaucoup aux dents".

Enfin, en ce qui concerne l'aromatisation du produit, 2 personnes ont trouvé le produit "mauvais", 6 l'ont trouvé "bon" et 8 l'ont trouvé "très bon".

Il ressort de ces différents essais que le produit conforme à l'invention est très bien perçu par les consommateurs sur un plan statistique.

## Revendications

1. Préparation solide vitamino-calcique comprenant du calcium et au moins une vitamine D, **caractérisée par le fait qu'**elle se présente sous la forme d'un comprimé à croquer susceptible d'être obtenu par compression directe et ayant une dureté comprise entre 75 et 300 N, de préférence entre 85 et 275 N, et plus préférentiellement encore entre 100 et 250 N et en ce qu'elle contient de 1 à 3 %, de préférence environ 2 % de lubrifiant, celui-ci étant préférentiellement le stéarate de magnésium.

2. Préparation solide vitamino-calcique selon la revendication 1, **caractérisée par le fait que** le calcium est amené par un sel choisi parmi le carbonate de calcium, le phosphate de calcium et/ou l'hydroxyapatite microcristalline, le carbonate de calcium étant préféré.

3. Préparation solide vitamino-calcique selon l'une ou l'autre des revendications 1 et 2, **caractérisé par le fait que** la vitamine D est choisie parmi la vitamine D2 ou la vitamine D3, la vitamine D3 étant préférée.

4. Préparation selon la revendication 3, **caractérisée par le fait que** la vitamine D3 est utilisée sous une forme pulvérulente incorporée dans une matrice qui augmente sa stabilité.

5. Préparation selon l'une quelconque des revendications 1 à 4, **caractérisée par le fait que** la quantité de sel de calcium dans le comprimé est comprise entre 500 et 2500 mg, de préférence entre 750 et 2000 mg et plus préférentiellement encore entre 1000 et 1500 mg.

6. Préparation selon l'une quelconque des revendications 1 à 5, **caractérisée par le fait que** la vitamine D dans le comprimé est comprise entre 50 et 1000 UI, de préférence entre 100 et 800 UI et plus préférentiellement encore entre 250 et 600 UI.

7. Préparation selon l'une quelconque des revendications 1 à 6, **caractérisée par le fait qu'**elle comprend au moins un excipient de compression directe, choisi de préférence parmi les polyols tels que le sorbitol, le xylitol, le maltitol et le mannitol.

8. Préparation selon l'une quelconque des revendications 1 à 7, **caractérisée par le fait que** l'excipient de compression est constitué par du sorbitol et du xylitol, le sorbitol constituant de préférence de 55 à 90 % et le xylitol constituant de préférence de 10 à 45 % dudit excipient.

9. Préparation selon l'une quelconque des revendications 1 à 8, **caractérisée par le fait qu'**elle contient au moins un arôme, celui-ci étant de préférence l'arôme cocktail de fruits IFF 34 A016.

10. Préparation selon l'une quelconque des revendications 1 à 9, **caractérisée par le fait qu'**elle présente la composition typique suivante :
- carbonate de calcium : 1100 - 1350 mg
- cholécalciférol (sous la forme de vitamine D3 enrobée) : 350 - 450 UI (3,5 - 4,5 mg)
- sorbitol : 750 - 1000 mg
- xylitol : 180 - 350 mg
- Aspartame : 4 - 8 mg
- Silice colloïdale anhydre : 8-15 mg
- Arôme : 40 - 70 mg
- Stéarate de magnésium : 45 - 80 mg.

11. Procédé de fabrication de la préparation solide vitamino-calcique selon l'une quelconque des revendications 1 à 10, **caractérisé par le fait que** l'on pèse les différents composants de la préparation, qu'on les mélange pendant un temps suffisant pour assurer une bonne homogénéisation, que l'on effectue la compression du mélange obtenu sur une machine à comprimer rotative ou alternative et que l'on conditionne les comprimés ainsi obtenus, les paramètres de compression étant choisis de façon à obtenir la dureté de comprimés voulue.

12. Utilisation de la préparation solide vitamino-calcique selon l'une quelconque des revendications 1 à 10 pour fabriquer un médicament destiné à prévenir et combattre l'ostéoporose.

## Claims

1. A solid vitamin-calcic preparation containing calcium and at least one vitamin D, **characterised in that** it is in the form of a tablet for chewing which is obtainable by direct compression and having a hardness between 75 and 300 N, preferably between 85 and 275 N, and still more preferably between 100 and 250 N and **in that** it contains 1 to 3%, preferably about 2%, of lubricant, the latter preferably being magnesium stearate.

2. A solid vitamin-calcic preparation according to claim 1, **characterised in that** the calcium is provided by a salt selected from calcium carbonate, calcium phosphate and/or microcrystalline hydroxyapatite, calcium carbonate being preferred.

3. A solid vitamin-calcic preparation according to clam 1 or 2, **characterised in that** the vitamin D is selected from vitamin D2 or vitamin D3, vitamin D3 being preferred.

4. A preparation according to claim 3, **characterised in that** the vitamin D3 is used in a powder form incorporated in a matrix which increases its stability.

5. A preparation according to any one of claims 1 to 4, **characterised in that** the quantity of calcium salt in the tablet is between 500 and 2500 mg, preferably between 750 and 2000 mg and still more preferably between 1000 and 1500 mg.

6. A preparation according to any one of claims 1 to 5, **characterised in that** the vitamin D in the tablet is between 50 and 1000 UI, preferably between 100 and 800 UI and still more preferably between 250 and 600 UI.

7. A preparation according to any one of claims 1 to 6, **characterised in that** it contains at least one direct compression excipient selected preferably among polyols such as sorbitol, xylitol, maltitol and mannitol.

8. A preparation according to any one of claims 1 to 7, **characterised in that** the compression excipient consists of sorbitol and xylitol, the sorbitol preferably being 55 to 90% and the xylitol preferably being 10 to 45% of the said excipient.

9. A preparation according to any one of claims 1 to 8, **characterised in that** it contains at least one flavouring, the latter preferably being IFF 34 A016 fruit cocktail flavouring.

10. A preparation according to any one of claims 1 to 9, **characterised in that** it has the following typical composition:
- calcium carbonate 1100-1.350 mg
- cholecalciferol (in the form of coated vitamin D3) 350- 450 UI
(3.5 - 4.5 mg)
- sorbitol 750 - 1000 mg
- xylitol 180 - 350 mg
- Aspartame 4 - 8 mg
- anhydrous colloidal silica 8 - 15 mg
- flavouring 40 - 60 mg
- magnesium stearate 45 - 80 mg

11. A process for the production of the solid vitamin-calcic preparation according to any one of claims 1 to 10, **characterised in that** the different components of the preparation are weighed, **in that** they are mixed for a period sufficient to ensure good homogenisation, **in that** compression of the resulting mixture is carried out on a rotary or reciprocating compression machine and **in that** the tablets thus produced are packed, the compression parameters being selected to give the desired tablet hardness.

12. Use of the solid vitamin-calcic preparatian according to any one of claims 1 to 10 to produce a medicament intended to prevent and combat osteoporosis.

## Patentansprüche

1. Feste Vitamin-Kalzium-Zubereitung, die Kalzium und zumindest ein Vitamin D enthält, **dadurch gekennzeichnet, daß** sie sich in der Form einer einzunehmenden Tablette darstellt, die durch direkte Pressung erlangbar ist und eine Härte zwischen 75 und 300 N, vorzugsweise zwischen 85 und 275 N und am besten noch zwischen 100 und 25 N hat, und daß sie 1 bis 3 %, vorzugsweise etwa 2 % Schmiermittel enthält, wobei dieses vorzugsweise Magnesium-stearat ist.

2. Feste Vitamin-Kaizium-Zubereitung nach Ansprüch 1, **dadurch gekennzeichnet, daß** das Kalzium durch ein Salz zugeführt wird, das aus Kalziumcarbonat, Kalziumphosphat und/oder mikrokristallinem Hydroxyapatit ausgewählt ist, wobei Kalziumcarbonat bevorzugt ist.

3. Feste Vitamin-Kalzium-Zubereitung nach einem der Ansprüche 1 und 2, **dadurch gekennzeichnet, daß** das Vitamin D aus dem Vitamin D2 oder dem Vitamin D3 ausgewählt wird, wobei das Vitamin D3 bevorzugt ist.

4. Zubereitung nach Ansprüch 3, **dadurch gekennzeichnet, daß** das Vitamin D3 in einer feinpulverigen Form benutzt wird, die in einer Matrix enthalten ist, die seine Stabilität steigert.

5. Zubereitung nach einem der Ansprüche 1 bis 4, **dadurch gekennzeichnet, daß** die Menge Kalziumsalz in der Tablette zwischen 500 und 2500 mg, vorzugsweise zwischen 750 und 2000 mg und am besten noch zwischen 1000 und 1500 mg liegt.

6. Zubereitung nach einem der Ansprüche 1 bis 5, **dadurch gekennzeichnet, daß** das Vitamin D in der Tablette zwischen 50 und 1000 I.E., vorzugsweise zwischen 100 und 800 I.E. und an besten noch zwischen 250 und 600 I.E. liegt.

7. Zubereitung nach einem der Ansprüche 1 bis 6, **dadurch gekennzeichnet, daß** sie wenigstens eine Grundmasse zur direkten Pressung umfaßt, die vorzugsweise aus den Polyolen, wie z.B. Sorbit, Xylit, Maltit und Mannit ausgewählt wird.

8. Zubereitung nach einem der Ansprüche 1 bis 7, **dadurch gekennzeichnet, daß** die Pressungsgrundmasse durch Sorbit und Xylit gebildet wird, wobei das Sortit vorzugsweise 55 bis 90 % und das Xylit vorzugsweise 10 bis 45 % der Grundmasse bilden.

9. Zubereitung nach einem der Ansprüche 1 bis 8, **dadurch gekennzeichnet, daß** sie wenigstens einen Geschmacksstoff enthält, wobei dieser vorzugsweise der Früchtecocktail-Geschmacksstoff IFF 34 A016 ist.

10. Zubereitung nach einem der Ansprüche 1 bis 9, **dadurch gekennzeichnet, daß** sie folgende typische Zusammensetzung aufweist:
- Kalziumcarbonat 1100 - 1350 mg
- Cholecalciferol (in Form von ummanteltem Vitamin D3) 350 - 450 I.E.
(3,5 - 4,5 mg)
- Sorbit 750 - 1000 mg
- Xylit 180 - 350 mg
- Aspartam 4 - 8 mg
- Wasserfreie Kolloidkieselsäure 8 - 15 mg
- Geschmacksstaff 40 - 70 mg
- Magnesiumstearat 45 - 80 mg

11. Verfahren zur Herstellung der festen Vitamin-Kalzium-Zubereitung nach einem der Ansprüche 1 bis 10, **dadurch gekennzeichnet, daß** die verschiedenen Komponenten der Zubereitung gewogen werden, daß sie während einer Zeitdauer gemischt werden, die ausreicht, um eine gute Homogenisierung sicherzustellen, daß die Pressung des erhaltenen Gemisches auf einer Rotations- oder alternativen Preßmaschine durchgeführt wird und daß die so erhaltenen Tabletten konditioniert werden, wobei die Preßparameter derart gewählt werden, daß die gewünschte Tablettenhärte erhalten wird.

12. Verwendung der festen Vitamin-Kalzium-Zubereitung nach einem der Ansprüche 1 bis 10 zum Herstellen eines Medikamentes, das zum Verhindern und Bekämpfen der Osteoporose bestimmt ist.
